# EUROPEAN PATENT APPLICATION

(11) **EP 1 614 754 A1**
(43) Date of publication of application: **11.01.2006**
(21) Application number: 04103188.1
(22) Date of filing: 06.07.2004
(51) Int. Cl.: C12N 15/82

(54) **Method for enhancing gene expression in plants**

(71) Applicant: Biogemma, 75001 Paris (FR)
(72) Inventor: The designation of the inventor has not yet been filed
(74) Representative: Flesselles, Bruno F.G.

(57) **Abstract**

The invention relates to the field of gene expression in plants and describes methods and constructs using the first intron of a FAD2 gene in order to enhance gene expression.

SEQUENCE LISTING

<110>  BIOGEMMA UK LIMITED

<120>  Method for enhancing gene expression in plants

<130>  BGM 29 - EP - PRIO

<160>  12

<170>  PatentIn version 3.2

<210>  1
<211>  1135
<212>  DNA
<213>  Arabidopsis thaliana

<400>  1
gtccgtcgct tctcttccat ttcttctcat tttcgatttt gattcttatt tctttccagt      60

agctcctgct ctgtgaattt ctccgctcac gatagatctg cttatactcc ttacattcaa     120

ccttagatct ggtctcgatt ctctgtttct ctgttttttt cttttggtcg agaatctgat     180

gtttgtttat gttctgtcac cattaataat aatgaactct ctcattcata caatgattag     240

tttctctcgt ctacaaaacg atatgttgca ttttcacttt tcttcttttt ttctaagatg     300

atttgctttg accaatttgt ttagatcttt attctatttt attttctggt gggttggtgg     360

aaattgaaaa aaaaaaaaac agcataaatt gttatttgtt aatgtattca ttttttggct     420

atttgttctg ggtaaaaatc tgcttctact attgaatctt tcctggattt tttactccta     480

ttgggttttt atagtaaaaa tacataataa aaggaaaaca aaagttttat agattctctt     540

aaacccctta cgataaaagt tggaatcaaa ataattcagg atcagatgct ctttgattga     600

ttcagatgcg attacagttg catggcaaat tttctagatc cgtcgtcaca ttttattttc     660

tgtttaaata tctaaatctg atatatgatg tcgatcgaca aattctggtg gcttatacat     720

cacttcaact gttttctttt ggctttgttt gtcaacttgg ttttcaatac gatttgtgat     780

ttcgatcgct gaatttttaa tacaagcaaa ctgatgttaa ccacaagcaa gagatgtgac     840

ctgccttatt aacatcgtat tacttactac tagtcgtatt ctcaacgcaa tcgtttttgt     900

atttctcaca ttatgccgct tctctactct ttattccttt tggtccacgc attttctatt     960

tgtggcaatc cctttcacaa cctgatttcc cactttggat catttgtctg aagactctct    1020

tgaatcgtta ccacttgttt cttgtgcatg ctctgttttt tagaattaat gataaaacta    1080

ttccatagtc ttgagttttc agcttgttga ttcttttgct tttggttttc tgcag         1135

<210>  2
<211>  3391
<212>  DNA
<213>  Artificial

<220>
<223>  proVir35Sx2 - EcGUS - terVirCaMV


<220>
<221>  promoter
<222>  (1)..(733)
<223>  35 S x 2

<220>
<221>  Coding_sequence
<222>  (782)..(2587)
<223>  GUS protein

<220>
<221>  misc_feature
<222>  (2645)..(2646)
<223>  n is a, c, g, or t

<220>
<221>  terminator
<222>  (2657)..(3391)

<400>  2
ctactccaaa aatgtcaaag atacagtctc agaagaccaa agggctattg agacttttca      60

acaaagggta atttcgggaa acctcctcgg attccattgc ccagctatct gtcacttcat     120

cgaaaggaca gtagaaaagg aaggtggctc ctacaaatgc catcattgcg ataaaggaaa     180

ggctatcatt caagatgcct ctgccgacag tggtcccaaa gatggacccc cacccacgag     240

gagcatcgtg gaaaaagaag acgttccaac cacgtcttca aagcaagtgg attgatgtga     300

catctccact gacgtaaggg atgacgcaca atcccacccc tactccaaaa atgtcaaaga     360

tacagtctca gaagaccaaa gggctattga gacttttcaa caaagggtaa tttcgggaaa     420

cctcctcgga ttccattgcc cagctatctg tcacttcatc gaaaggacag tagaaaagga     480

aggtggctcc tacaaatgcc atcattgcga taaaggaaag gctatcattc aagatgcctc     540

tgccgacagt ggtcccaaag atggaccccc acccacgagg agcatcgtgg aaaaagaaga     600

cgttccaacc acgtcttcaa agcaagtgga ttgatgtgac atctccactg acgtaaggga     660

tgacgcacaa tcccactatc cttcgcaaga cccttcctct atataaggaa gttcatttca     720

tttggagagg acagcccaag cttggctgca ggtcgacgga tccccgggtg gtcagtccct     780

tatgttacgt cctgtagaaa ccccaacccg tgaaatcaaa aaactcgacg gcctgtgggc     840

attcagtctg gatcgcgaaa actgtggaat tgatcagcgt tggtgggaaa gcgcgttaca     900

agaaagccgg gcaattgctg tgccaggcag ttttaacgat cagttcgccg atgcagatat     960

tcgtaattat gcgggcaacg tctggtatca gcgcgaagtc tttataccga aaggttgggc    1020

aggccagcgt atcgtgctgc gtttcgatgc ggtcactcat tacggcaaag tgtgggtcaa    1080

taatcaggaa gtgatggagc atcagggcgg ctatacgcca tttgaagccg atgtcacgcc 1140

gtatgttatt gccgggaaaa gtgtacgtat caccgtttgt gtgaacaacg aactgaactg 1200

gcagactatc ccgccgggaa tggtgattac cgacgaaaac ggcaagaaaa agcagtctta 1260

cttccatgat ttctttaact atgccggaat ccatcgcagc gtaatgctct acaccacgcc 1320

gaacacctgg gtggacgata tcaccgtggt gacgcatgtc gcgcaagact gtaaccacgc 1380

gtctgttgac tggcaggtgg tggccaatgg tgatgtcagc gttgaactgc gtgatgcgga 1440

tcaacaggtg gttgcaactg gacaaggcac tagcgggact ttgcaagtgg tgaatccgca 1500

cctctggcaa ccgggtgaag gttatctcta tgaactgtgc gtcacagcca aaagccagac 1560

agagtgtgat atctacccgc ttcgcgtcgg catccggtca gtggcagtga agggccaaca 1620

gttcctgatt aaccacaaac cgttctactt tactggcttt ggtcgtcatg aagatgcgga 1680

cttacgtggc aaaggattcg ataacgtgct gatggtgcac gaccacgcat taatggactg 1740

gattggggcc aactcctacc gtacctcgca ttacccttac gctgaagaga tgctcgactg 1800

ggcagatgaa catggcatcg tggtgattga tgaaactgct gctgtcggct ttaacctctc 1860

tttaggcatt ggtttcgaag cgggcaacaa gccgaaagaa ctgtacagcg aagaggcagt 1920

caacggggaa actcagcaag cgcacttaca ggcgattaaa gagctgatag cgcgtgacaa 1980

aaaccaccca agcgtggtga tgtggagtat tgccaacgaa ccggataccc gtccgcaagt 2040

gcacgggaat atttcgccac tggcggaagc aacgcgtaaa ctcgacccga cgcgtccgat 2100

cacctgcgtc aatgtaatgt tctgcgacgc tcacaccgat accatcagcg atctctttga 2160

tgtgctgtgc ctgaaccgtt attacggatg gtatgtccaa agcggcgatt tggaaacggc 2220

agagaaggta ctggaaaaag aacttctggc ctggcaggag aaactgcatc agccgattat 2280

catcaccgaa tacggcgtgg atacgttagc cgggctgcac tcaatgtaca ccgacatgtg 2340

gagtgaagag tatcagtgtg catggctgga tatgtatcac cgcgtctttg atcgcgtcag 2400

cgccgtcgtc ggtgaacagg tatggaattt cgccgatttt gcgacctcgc aaggcatatt 2460

gcgcgttggc ggtaacaaga aagggatctt cactcgcgac cgcaaaccga agtcggcggc 2520

ttttctgctg caaaaacgct ggactggcat gaacttcggt gaaaaaccgc agcagggagg 2580

caaacaatga atcaacaact ctcctggcgc accatcgtcg gctacagcct cgggaattgc 2640

taccnntcta gaattcggct gaaatcacca gtctctctct acaaatctat ctctctctat 2700

tttctccata aataatgtgt gagtagtttc ccgataaggg aaattagggt tcttataggg 2760

tttcgctcat gtgttgagca tataagaaac ccttagtatg tatttgtatt tgtaaaatac 2820

ttctatcaat aaaatttcta attcctaaaa ccaaaatcca gtactaaaat ccagatctcc 2880

taaagtccct atagatcttt gtcgtgaata taaaccagac acgagacgac taaacctgga 2940

gcccagacgc cgttcgaagc tagaagtacc gcttaggcag gaggccgtta gggaaaagat 3000

gctaaggcag ggttggttac gttgactccc ccgtaggttt ggtttaaata tgatgaagtg 3060

gacggaagga aggaggaaga caaggaagga taaggttgca ggccctgtgc aaggtaagaa 3120

gatggaaatt tgatagaggt acgctactat acttatacta tacgctaagg gaatgcttgt 3180

atttataccc tataccccct aataacccct tatcaattta agaaataatc cgcataagcc 3240

cccgcttaaa aattggtatc agagccatga ataggtctat gaccaaaact caagaggata 3300

aaacctcacc aaaatacgaa agagttctta actctaaaga taaaagatct ttcaagatca 3360

aaactagttc cctcacaccg gtgacgggga t 3391

<210> 3
<211> 4534
<212> DNA
<213> artificial

<220>
<223> proVir35Sx2 - intAtFAD2 - EcGUS - terVirCaMV

<220>
<221> promoter
<222> (1)..(733)
<223> 35 S x 2

<220>
<221> Intron
<222> (772)..(1902)
<223> FAD 2 intron

<220>
<221> Coding_sequence
<222> (1925)..(3730)
<223> GUS protein

<220>
<221> misc_feature
<222> (3788)..(3789)
<223> n is a, c, g, or t

<220>
<221> terminator
<222> (3800)..(4534)
<223> CAMV terminator

<400> 3
ctactccaaa aatgtcaaag atacagtctc agaagaccaa agggctattg agacttttca 60

acaaagggta atttcgggaa acctcctcgg attccattgc ccagctatct gtcacttcat 120

cgaaaggaca gtagaaaagg aaggtggctc ctacaaatgc catcattgcg ataaaggaaa 180

ggctatcatt caagatgcct ctgccgacag tggtcccaaa gatggacccc cacccacgag 240

gagcatcgtg gaaaaagaag acgttccaac cacgtcttca aagcaagtgg attgatgtga 300

catctccact gacgtaaggg atgacgcaca atcccacccc tactccaaaa atgtcaaaga 360

tacagtctca gaagaccaaa gggctattga gacttttcaa caaagggtaa tttcgggaaa 420

cctcctcgga ttccattgcc cagctatctg tcacttcatc gaaaggacag tagaaaagga 480

aggtggctcc tacaaatgcc atcattgcga taaaggaaag gctatcattc aagatgcctc 540

tgccgacagt ggtcccaaag atggaccccc acccacgagg agcatcgtgg aaaaagaaga 600

cgttccaacc acgtcttcaa agcaagtgga ttgatgtgac atctccactg acgtaaggga 660

tgacgcacaa tcccactatc cttcgcaaga cccttcctct atataaggaa gttcatttca 720

tttggagagg acagcccaag cttggctgca ggtcgacgga tccccgggtt tgtccgtcgc 780

ttctcttcca tttcttctca ttttcgattt tgattcttat ttctttccag tagctcctgc 840

tctgtgaatt tctccgctca cgatagatct gcttatactc cttacattca accttagatc 900

tggtctcgat tctctgtttc tctgtttttt tcttttggtc gagaatctga tgtttgttta 960

tgttctgtca ccattaataa taatgaactc tctcattcat acaatgatta gtttctctcg 1020

tctacaaaac gatatgttgc attttcactt ttcttctttt tttctaagat gatttgcttt 1080

gaccaatttg tttagatctt tattctattt tattttctgg tgggttggtg gaaattgaaa 1140

aaaaaaaaaa cagcataaat tgttatttgt taatgtattc attttttggc tatttgttct 1200

gggtaaaaat ctgcttctac tattgaatct ttcctggatt ttttactcct attgggtttt 1260

tatagtaaaa atacataata aaaggaaaac aaaagtttta tagattctct taaacccctt 1320

acgataaaag ttggaatcaa aataattcag gatcagatgc tctttgattg attcagatgc 1380

gattacagtt gcatggcaaa ttttctagat ccgtcgtcac attttatttt ctgtttaaat 1440

atctaaatct gatatatgat gtcgacaaat tctggtggct tatacatcac ttcaactgtt 1500

ttcttttggc tttgtttgtc aacttggttt tcaatacgat ttgtgatttc gatcgctgaa 1560

tttttaatac aagcaaactg atgttaacca caagcaagag atgtgacctg ccttattaac 1620

atcgtattac ttactactag tcgtattctc aacgcaatcg tttttgtatt tctcacatta 1680

tgccgcttct ctactcttta ttccttttgg tccacgcatt ttctatttgt ggcaatccct 1740

ttcacaacct gatttcccac tttggatcat ttgtctgaag actctcttga atcgttacca 1800

cttgtttctt gtgcatgctc tgttttttag aattaatgat aaaactattc catagtcttg 1860

agttttcagc ttgttgattc ttttgctttt ggttttctgc agtggcccgg gtggtcagtc 1920

ccttatgtta cgtcctgtag aaaccccaac ccgtgaaatc aaaaaactcg acggcctgtg 1980

ggcattcagt ctggatcgcg aaaactgtgg aattgatcag cgttggtggg aaagcgcgtt 2040

acaagaaagc cgggcaattg ctgtgccagg cagttttaac gatcagttcg ccgatgcaga 2100

tattcgtaat tatgcgggca acgtctggta tcagcgcgaa gtctttatac cgaaaggttg 2160

ggcaggccag cgtatcgtgc tgcgtttcga tgcggtcact cattacggca aagtgtgggt 2220

caataatcag gaagtgatgg agcatcaggg cggctatacg ccatttgaag ccgatgtcac 2280

gccgtatgtt attgccggga aaagtgtacg tatcaccgtt tgtgtgaaca acgaactgaa 2340

ctggcagact atcccgccgg gaatggtgat taccgacgaa aacggcaaga aaaagcagtc 2400

ttacttccat gatttcttta actatgccgg aatccatcgc agcgtaatgc tctacaccac 2460

gccgaacacc tgggtggacg atatcaccgt ggtgacgcat gtcgcgcaag actgtaacca 2520

cgcgtctgtt gactggcagg tggtggccaa tggtgatgtc agcgttgaac tgcgtgatgc 2580

ggatcaacag gtggttgcaa ctggacaagg cactagcggg actttgcaag tggtgaatcc 2640

gcacctctgg caaccgggtg aaggttatct ctatgaactg tgcgtcacag ccaaaagcca 2700

gacagagtgt gatatctacc cgcttcgcgt cggcatccgg tcagtggcag tgaagggcca 2760

acagttcctg attaaccaca aaccgttcta ctttactggc tttggtcgtc atgaagatgc 2820

ggacttacgt ggcaaaggat tcgataacgt gctgatggtg cacgaccacg cattaatgga 2880

ctggattggg gccaactcct accgtacctc gcattaccct tacgctgaag agatgctcga 2940

ctgggcagat gaacatggca tcgtggtgat tgatgaaact gctgctgtcg gctttaacct 3000

ctctttaggc attggtttcg aagcgggcaa caagccgaaa gaactgtaca gcgaagaggc 3060

agtcaacggg gaaactcagc aagcgcactt acaggcgatt aaagagctga tagcgcgtga 3120

caaaaaccac ccaagcgtgg tgatgtggag tattgccaac gaaccggata cccgtccgca 3180

agtgcacggg aatatttcgc cactggcgga agcaacgcgt aaactcgacc cgacgcgtcc 3240

gatcacctgc gtcaatgtaa tgttctgcga cgctcacacc gataccatca gcgatctctt 3300

tgatgtgctg tgcctgaacc gttattacgg atggtatgtc caaagcggcg atttggaaac 3360

ggcagagaag gtactggaaa aagaacttct ggcctggcag gagaaactgc atcagccgat 3420

tatcatcacc gaatacggcg tggatacgtt agccgggctg cactcaatgt acaccgacat 3480

gtggagtgaa gagtatcagt gtgcatggct ggatatgtat caccgcgtct ttgatcgcgt 3540

cagcgccgtc gtcggtgaac aggtatggaa tttcgccgat tttgcgacct cgcaaggcat 3600

attgcgcgtt ggcggtaaca agaaagggat cttcactcgc gaccgcaaac cgaagtcggc 3660

ggcttttctg ctgcaaaaac gctggactgg catgaacttc ggtgaaaaac cgcagcaggg 3720

aggcaaacaa tgaatcaaca actctcctgg cgcaccatcg tcggctacag cctcgggaat 3780

tgctaccnnt ctagaattcg gctgaaatca ccagtctctc tctacaaatc tatctctctc 3840

tattttctcc ataaataatg tgtgagtagt ttcccgataa gggaaattag ggttcttata 3900

gggtttcgct catgtgttga gcatataaga aacccttagt atgtatttgt atttgtaaaa 3960

tacttctatc aataaaattt ctaattccta aaaccaaaat ccagtactaa aatccagatc 4020

tcctaaagtc cctatagatc tttgtcgtga atataaacca gacacgagac gactaaacct 4080

ggagcccaga cgccgttcga agctagaagt accgcttagg caggaggccg ttagggaaaa 4140

gatgctaagg cagggttggt tacgttgact cccccgtagg tttggtttaa atatgatgaa 4200

gtggacggaa ggaaggagga agacaaggaa ggataaggtt gcaggccctg tgcaaggtaa 4260

gaagatggaa atttgataga ggtacgctac tatacttata ctatacgcta agggaatgct 4320

tgtatttata ccctataccc cctaataacc ccttatcaat ttaagaaata atccgcataa 4380

gcccccgctt aaaaattggt atcagagcca tgaataggtc tatgaccaaa actcaagagg 4440

ataaaacctc accaaaatac gaaagagttc ttaactctaa agataaaaga tctttcaaga 4500

tcaaaactag ttccctcaca ccggtgacgg ggat 4534

<210> 4
<211> 3361
<212> DNA
<213> artificial

<220>
<223> proVirSc4 - EcGUSintStLS1 - terVirCaMV

<220>
<221> promoter
<222> (1)..(532)
<223> Sc4 promoter

<220>
<221> Coding_sequence
<222> (551)..(2545)
<223> Gus protein

<220>
<221> terminator
<222> (2578)..(3361)
<223> CAMV terminator

<400> 4
taattgttat tatcaataaa agaattttta ttgttattgt gttatttggt aatttatgct 60

tataagtaat tctatgatta attgtgaatt aataagacta atgaggataa taattgaatt 120

tgattaaatt aactctgcga agccatatgt ctttcacgtg agagtcacgt gatgtctccg 180

cgacaggctg gcacggggct tagtattacc cccgtgccgg gatcagagac atttgactaa 240

atgttgactt ggaataatag cccttggatt agatgacacg tggacgctca ggatctgtga 300

tgctagtgaa gcgcttaagc tgaacgaatc tgacggaaga gcggacaaac gcacatggac 360

tatggcccac tgctttatta aagaagtgaa tgacagctgt ctttgcttca agacgaagta 420

aagaatagtg gaaaacgcgt aaagaataag cgtactcagt acgcttcgtg gctttataaa 480

tagtgcttcg tcttattctt cgttgtatca tcaacgaaga agttaagctg atcccgggaa 540

ttcgcggccc atggtacgtc ctgtagaaac cccaacccgt gaaatcaaaa aactcgacgg 600

cctgtgggca ttcagtctgg atcgcgaaaa ctgtggaatt gatcagcgtt ggtgggaaag 660

cgcgttacaa gaaagccggg caattgctgt gccaggcagt tttaacgatc agttcgccga 720

tgcagatatt cgtaattatg cgggcaacgt ctggtatcag cgcgaagtct ttataccgaa 780

aggttgggca ggccagcgta tcgtgctgcg tttcgatgcg gtcactcatt acggcaaagt 840

gtgggtcaat aatcaggaag tgatggagca tcagggcggc tatacgccat ttgaagccga 900

tgtcacgccg tatgttattg ccgggaaaag tgtacgtaag tttctgcttc tacctttgat 960

atatatataa taattatcat taattagtag taatataata tttcaaatat ttttttcaaa 1020

ataaaagaat gtagtatata gcaattgctt ttctgtagtt tataagtgtg tatattttaa 1080

tttataactt ttctaatata tgaccaaaat ttgttgatgt gcaggtatca ccgtttgtgt 1140

gaacaacgaa ctgaactggc agactatccc gccgggaatg gtgattaccg acgaaaacgg 1200

caagaaaaag cagtcttact tccatgattt ctttaactat gccggaatcc atcgcagcgt 1260

aatgctctac accacgccga acacctgggt ggacgatatc accgtggtga cgcatgtcgc 1320

gcaagactgt aaccacgcgt ctgttgactg gcaggtggtg gccaatggtg atgtcagcgt 1380

tgaactgcgt gatgcggatc aacaggtggt tgcaactgga caaggcacta gcgggacttt 1440

gcaagtggtg aatccgcacc tctggcaacc gggtgaaggt tatctctatg aactgtgcgt 1500

cacagccaaa agccagacag agtgtgatat ctacccgctt cgcgtcggca tccggtcagt 1560

ggcagtgaag ggccaacagt tcctgattaa ccacaaaccg ttctacttta ctggctttgg 1620

tcgtcatgaa gatgcggact tacgtggcaa aggattcgat aacgtgctga tggtgcacga 1680

ccacgcatta atggactgga ttggggccaa ctcctaccgt acctcgcatt acccttacgc 1740

tgaagagatg ctcgactggg cagatgaaca tggcatcgtg gtgattgatg aaactgctgc 1800

tgtcggcttt aacctctctt taggcattgg tttcgaagcg ggcaacaagc cgaaagaact 1860

gtacagcgaa gaggcagtca acggggaaac tcagcaagcg cacttacagg cgattaaaga 1920

gctgatagcg cgtgacaaaa accacccaag cgtggtgatg tggagtattg ccaacgaacc 1980

ggatacccgt ccgcaagtgc acgggaatat ttcgccactg gcggaagcaa cgcgtaaact 2040

cgacccgacg cgtccgatca cctgcgtcaa tgtaatgttc tgcgacgctc acaccgatac 2100

catcagcgat ctctttgatg tgctgtgcct gaaccgttat tacggatggt atgtccaaag 2160

cggcgatttg gaaacggcag agaaggtact ggaaaaagaa cttctggcct ggcaggagaa 2220

actgcatcag ccgattatca tcaccgaata cggcgtggat acgttagccg ggctgcactc 2280

aatgtacacc gacatgtgga gtgaagagta tcagtgtgca tggctggata tgtatcaccg 2340

cgtctttgat cgcgtcagcg ccgtcgtcgg tgaacaggta tggaatttcg ccgattttgc 2400

gacctcgcaa ggcatattgc gcgttggcgg taacaagaaa gggatcttca ctcgcgaccg 2460

caaaccgaag tcggcggctt ttctgctgca aaaacgctgg actggcatga acttcggtga 2520

aaaaccgcag cagggaggca aacaatgaat caacaactct cctggcgcac catcgtcggc 2580

tacagcctcg gtgacgtcgg atcccccggg ctgcaggaat tcggtacgct gaaatcacca 2640

gtctctctct acaaatctat ctctctctat tttctccata aataatgtgt gagtagtttc 2700

ccgataaggg aaattagggt tcttataggg tttcgctcat gtgttgagca tataagaaac 2760

ccttagtatg tatttgtatt tgtaaaatac ttctatcaat aaaatttcta attcctaaaa 2820

ccaaaatcca gtactaaaat ccagatctcc taaagtccct atagatcttt gtcgtgaata 2880

taaaccagac acgagacgac taaacctgga gcccagacgc cgttcgaagc tagaagtacc 2940

gcttaggcag gaggccgtta gggaaaagat gctaaggcag ggttggttac gttgactccc 3000

ccgtaggttt ggtttaaata tgatgaagtg gacggaagga aggaggaaga caaggaagga 3060

taaggttgca ggccctgtgc aaggtaagaa gatggaaatt tgatagaggt acgctactat 3120

acttatacta tacgctaagg gaatgcttgt atttataccc tataccccct aataacccct 3180

tatcaattta agaaataatc cgcataagcc cccgcttaaa aattggtatc agagccatga 3240

ataggtctat gaccaaaact caagaggata aaacctcacc aaaatacgaa agagttctta 3300

actctaaaga taaaagatct ttcaagatca aaactagttc cctcacaccg gtgacgggga 3360

t 3361

<210> 5
<211> 4561
<212> DNA
<213> artificial

<220>
<223> proVirSc4 - intAtFAD2 - EcGUSintStLS1 - terVirCaMV


<220>
<221> promoter
<222> (1)..(532)
<223> Sc4 promoter

<220>
<221> Intron
<222> (580)..(1714)
<223> FAD 2 intron

<220>

<221>  Coding_sequence
<222>  (1751)..(3745)
<223>  GUS protein

<220>
<221>  terminator
<222>  (3778)..(4561)
<223>  CAMV terminator

<400>  5
taattgttat tatcaataaa agaattttta ttgttattgt gttatttggt aatttatgct      60

tataagtaat tctatgatta attgtgaatt aataagacta atgaggataa taattgaatt     120

tgattaaatt aactctgcga agccatatgt ctttcacgtg agagtcacgt gatgtctccg     180

cgacaggctg gcacggggct tagtattacc cccgtgccgg gatcagagac atttgactaa     240

atgttgactt ggaataatag cccttggatt agatgacacg tggacgctca ggatctgtga     300

tgctagtgaa gcgcttaagc tgaacgaatc tgacggaaga gcggacaaac gcacatggac     360

tatggcccac tgctttatta aagaagtgaa tgacagctgt ctttgcttca agacgaagta     420

aagaatagtg gaaaacgcgt aaagaataag cgtactcagt acgcttcgtg gctttataaa     480

tagtgcttcg tcttattctt cgttgtatca tcaacgaaga agttaagctg atcccgggaa     540

ttcgcggccg ctctagaact agtggatccc ccgggccagg tccgtcgctt ctcttccatt     600

tcttctcatt ttcgattttg attcttattt ctttccagta gctcctgctc tgtgaatttc     660

tccgctcacg atagatctgc ttatactcct tacattcaac cttagatctg gtctcgattc     720

tctgtttctc tgtttttttc ttttggtcga gaatctgatg tttgtttatg ttctgtcacc     780

attaataata atgaactctc tcattcatac aatgattagt ttctctcgtc tacaaaacga     840

tatgttgcat tttcactttt cttctttttt tctaagatga tttgctttga ccaatttgtt     900

tagatcttta ttctatttta ttttctggtg ggttggtgga aattgaaaaa aaaaaaaaca     960

gcataaattg ttatttgtta atgtattcat tttttggcta tttgttctgg gtaaaaatct    1020

gcttctacta ttgaatcttt cctggatttt ttactcctat tgggttttta tagtaaaaat    1080

acataataaa aggaaaacaa aagttttata gattctctta aaccccttac gataaaagtt    1140

ggaatcaaaa taattcagga tcagatgctc tttgattgat tcagatgcga ttacagttgc    1200

atggcaaatt ttctagatcc gtcgtcacat tttattttct gtttaaatat ctaaatctga    1260

tatatgatgt cgatcgacaa attctggtgg cttatacatc acttcaactg ttttcttttg    1320

gctttgtttg tcaacttggt tttcaatacg atttgtgatt tcgatcgctg aatttttaat    1380

acaagcaaac tgatgttaac cacaagcaag agatgtgacc tgccttatta acatcgtatt    1440

acttactact agtcgtattc tcaacgcaat cgtttttgta tttctcacat tatgccgctt    1500

ctctactctt tattcctttt ggtccacgca ttttctattt gtggcaatcc ctttcacaac    1560

ctgatttccc actttggatc atttgtctga agactctctt gaatcgttac cacttgtttc 1620

ttgtgcatgc tctgtttttt agaattaatg ataaaactat tccatagtct tgagttttca 1680

gcttgttgat tcttttgctt ttggttttct gcagaaaccc gggctgcagg aattcgatat 1740

caagcttccc atggtacgtc ctgtagaaac cccaacccgt gaaatcaaaa aactcgacgg 1800

cctgtgggca ttcagtctgg atcgcgaaaa ctgtggaatt gatcagcgtt ggtgggaaag 1860

cgcgttacaa gaaagccggg caattgctgt gccaggcagt tttaacgatc agttcgccga 1920

tgcagatatt cgtaattatg cgggcaacgt ctggtatcag cgcgaagtct ttataccgaa 1980

aggttgggca ggccagcgta tcgtgctgcg tttcgatgcg gtcactcatt acggcaaagt 2040

gtgggtcaat aatcaggaag tgatggagca tcagggcggc tatacgccat ttgaagccga 2100

tgtcacgccg tatgttattg ccgggaaaag tgtacgtaag tttctgcttc tacctttgat 2160

atatatataa taattatcat taattagtag taatataata tttcaaatat ttttttcaaa 2220

ataaaagaat gtagtatata gcaattgctt ttctgtagtt tataagtgtg tatattttaa 2280

tttataactt ttctaatata tgaccaaaat ttgttgatgt gcaggtatca ccgtttgtgt 2340

gaacaacgaa ctgaactggc agactatccc gccgggaatg gtgattaccg acgaaaacgg 2400

caagaaaaag cagtcttact tccatgattt ctttaactat gccggaatcc atcgcagcgt 2460

aatgctctac accacgccga acacctgggt ggacgatatc accgtggtga cgcatgtcgc 2520

gcaagactgt aaccacgcgt ctgttgactg gcaggtggtg gccaatggtg atgtcagcgt 2580

tgaactgcgt gatgcggatc aacaggtggt tgcaactgga caaggcacta gcgggacttt 2640

gcaagtggtg aatccgcacc tctggcaacc gggtgaaggt tatctctatg aactgtgcgt 2700

cacagccaaa agccagacag agtgtgatat ctacccgctt cgcgtcggca tccggtcagt 2760

ggcagtgaag ggccaacagt tcctgattaa ccacaaaccg ttctacttta ctggctttgg 2820

tcgtcatgaa gatgcggact tacgtggcaa aggattcgat aacgtgctga tggtgcacga 2880

ccacgcatta atggactgga ttggggccaa ctcctaccgt acctcgcatt acccttacgc 2940

tgaagagatg ctcgactggg cagatgaaca tggcatcgtg gtgattgatg aaactgctgc 3000

tgtcggcttt aacctctctt taggcattgg tttcgaagcg ggcaacaagc cgaaagaact 3060

gtacagcgaa gaggcagtca acggggaaac tcagcaagcg cacttacagg cgattaaaga 3120

gctgatagcg cgtgacaaaa accacccaag cgtggtgatg tggagtattg ccaacgaacc 3180

ggatacccgt ccgcaagtgc acgggaatat ttcgccactg gcggaagcaa cgcgtaaact 3240

cgacccgacg cgtccgatca cctgcgtcaa tgtaatgttc tgcgacgctc acaccgatac 3300

catcagcgat ctctttgatg tgctgtgcct gaaccgttat tacggatggt atgtccaaag 3360

cggcgatttg gaaacggcag agaaggtact ggaaaaagaa cttctggcct ggcaggagaa 3420

actgcatcag ccgattatca tcaccgaata cggcgtggat acgttagccg ggctgcactc 3480

aatgtacacc gacatgtgga gtgaagagta tcagtgtgca tggctggata tgtatcaccg 3540

cgtctttgat cgcgtcagcg ccgtcgtcgg tgaacaggta tggaatttcg ccgattttgc 3600

gacctcgcaa ggcatattgc gcgttggcgg taacaagaaa gggatcttca ctcgcgaccg 3660

caaaccgaag tcggcggctt ttctgctgca aaaacgctgg actggcatga acttcggtga 3720

aaaaccgcag cagggaggca aacaatgaat caacaactct cctggcgcac catcgtcggc 3780

tacagcctcg gtgacgtcgg atcccccggg ctgcaggaat tcggtacgct gaaatcacca 3840

gtctctctct acaaatctat ctctctctat tttctccata aataatgtgt gagtagtttc 3900

ccgataaggg aaattagggt tcttataggg tttcgctcat gtgttgagca tataagaaac 3960

ccttagtatg tatttgtatt tgtaaaatac ttctatcaat aaaatttcta attcctaaaa 4020

ccaaaatcca gtactaaaat ccagatctcc taaagtccct atagatcttt gtcgtgaata 4080

taaaccagac acgagacgac taaacctgga gcccagacgc cgttcgaagc tagaagtacc 4140

gcttaggcag gaggccgtta gggaaaagat gctaaggcag ggttggttac gttgactccc 4200

ccgtaggttt ggtttaaata tgatgaagtg gacggaagga aggaggaaga caaggaagga 4260

taaggttgca ggccctgtgc aaggtaagaa gatggaaatt tgatagaggt acgctactat 4320

acttatacta tacgctaagg gaatgcttgt atttataccc tataccccct aataacccct 4380

tatcaattta agaaataatc cgcataagcc cccgcttaaa aattggtatc agagccatga 4440

ataggtctat gaccaaaact caagaggata aaacctcacc aaaatacgaa agagttctta 4500

actctaaaga taaaagatct ttcaagatca aaactagttc cctcacaccg gtgacgggga 4560

t 4561

<210> 6
<211> 3307
<212> DNA
<213> artificial

<220>
<223> proVirBSV - EcGUSintStLS1 - terVirCaMV

<220>
<221> promoter
<222> (1)..(494)
<223> BSV promoter

<220>
<221> Coding_sequence
<222> (497)..(2491)
<223> GUS protein

<220>
<221>  terminator
<222>  (2571)..(3307)
<223>  CAMV terminator

<400>  6
gatgctctag atctgctgga tatcagtaat gatgactgaa gcggaagtgg cggaccccta      60

ccacgtgttg ataccaaccg gtgtgaagac tgataagatg cggagtgagc tggataccac     120

tcactttatg taaagaggag acaaagtata atgtctcttt attttaagtt tgtcggtgtg     180

cgttgtctag tcacgcacga tgacctttag tgactttgca ggattcttac gcaaagttgt     240

taggccagag acatgtgatg atgcttatct gcattattgg tggatgccac ctaacgatgc     300

cagaaagctc cacaactctc tatataagga gccttgtatt caggttgcaa acacgcacca     360

caacgcgagt ttactcctga tttgagaaat aaaaacttct gtgcttgaaa cacactttgt     420

gcgagttcac tttgtgcgag tagagcgcaa gatcctagtt ccgcgagcgt agacccgtct     480

ggtatcagag caagccatgg tacgtcctgt agaaacccca acccgtgaaa tcaaaaaact     540

cgacggcctg tgggcattca gtctggatcg cgaaaactgt ggaattgatc agcgttggtg     600

ggaaagcgcg ttacaagaaa gccgggcaat tgctgtgcca ggcagtttta acgatcagtt     660

cgccgatgca gatattcgta attatgcggg caacgtctgg tatcagcgcg aagtctttat     720

accgaaaggt tgggcaggcc agcgtatcgt gctgcgtttc gatgcggtca ctcattacgg     780

caaagtgtgg gtcaataatc aggaagtgat ggagcatcag ggcggctata cgccatttga     840

agccgatgtc acgccgtatg ttattgccgg gaaaagtgta cgtaagtttc tgcttctacc     900

tttgatatat atataataat tatcattaat tagtagtaat ataatatttc aaatattttt     960

ttcaaaataa aagaatgtag tatatagcaa ttgcttttct gtagtttata agtgtgtata    1020

ttttaattta taacttttct aatatatgac caaaatttgt tgatgtgcag gtatcaccgt    1080

ttgtgtgaac aacgaactga actggcagac tatcccgccg ggaatggtga ttaccgacga    1140

aaacggcaag aaaaagcagt cttacttcca tgatttcttt aactatgccg gaatccatcg    1200

cagcgtaatg ctctacacca cgccgaacac ctgggtggac gatatcaccg tggtgacgca    1260

tgtcgcgcaa gactgtaacc acgcgtctgt tgactggcag gtggtggcca atggtgatgt    1320

cagcgttgaa ctgcgtgatg cggatcaaca ggtggttgca actggacaag gcactagcgg    1380

gactttgcaa gtggtgaatc cgcacctctg gcaaccgggt gaaggttatc tctatgaact    1440

gtgcgtcaca gccaaaagcc agacagagtg tgatatctac ccgcttcgcg tcggcatccg    1500

gtcagtggca gtgaagggcc aacagttcct gattaaccac aaaccgttct actttactgg    1560

ctttggtcgt catgaagatg cggacttacg tggcaaagga ttcgataacg tgctgatggt    1620

gcacgaccac gcattaatgg actggattgg ggccaactcc taccgtacct cgcattaccc 1680

ttacgctgaa gagatgctcg actgggcaga tgaacatggc atcgtggtga ttgatgaaac 1740

tgctgctgtc ggctttaacc tctctttagg cattggtttc gaagcgggca acaagccgaa 1800

agaactgtac agcgaagagg cagtcaacgg ggaaactcag caagcgcact tacaggcgat 1860

taaagagctg atagcgcgtg acaaaaacca cccaagcgtg gtgatgtgga gtattgccaa 1920

cgaaccggat acccgtccgc aagtgcacgg gaatatttcg ccactggcgg aagcaacgcg 1980

taaactcgac ccgacgcgtc cgatcacctg cgtcaatgta atgttctgcg acgctcacac 2040

cgataccatc agcgatctct ttgatgtgct gtgcctgaac cgttattacg gatggtatgt 2100

ccaaagcggc gatttggaaa cggcagagaa ggtactggaa aaagaacttc tggcctggca 2160

ggagaaactg catcagccga ttatcatcac cgaatacggc gtggatacgt tagccgggct 2220

gcactcaatg tacaccgaca tgtggagtga agagtatcag tgtgcatggc tggatatgta 2280

tcaccgcgtc tttgatcgcg tcagcgccgt cgtcggtgaa caggtatgga atttcgccga 2340

ttttgcgacc tcgcaaggca tattgcgcgt tggcggtaac aagaaaggga tcttcactcg 2400

cgaccgcaaa ccgaagtcgg cggcttttct gctgcaaaaa cgctggactg gcatgaactt 2460

cggtgaaaaa ccgcagcagg gaggcaaaca atgaatcaac aactctcctg gcgcaccatc 2520

gtcggctaca gcctcggtga cgtcggatcc cccgggctgc aggaattcgg tacgctgaaa 2580

tcaccagtct ctctctacaa atctatctct ctctattttc tccataaata atgtgtgagt 2640

agtttcccga taagggaaat tagggttctt atagggtttc gctcatgtgt tgagcatata 2700

agaaaccctt agtatgtatt tgtatttgta aaatacttct atcaataaaa tttctaattc 2760

ctaaaaccaa aatccagtac taaaatccag atctcctaaa gtccctatag atctttgtcg 2820

tgaatataaa ccagacacga gacgactaaa cctggagccc agacgccgtt cgaagctaga 2880

agtaccgctt aggcaggagg ccgttaggga aaagatgcta aggcagggtt ggttacgttg 2940

actcccccgt aggtttggtt taaatatgat gaagtggacg gaaggaagga ggaagacaag 3000

gaaggataag gttgcaggcc ctgtgcaagg taagaagatg gaaatttgat agaggtacgc 3060

tactatactt atactatacg ctaagggaat gcttgtattt ataccctata ccccctaata 3120

accccttatc aatttaagaa ataatccgca taagcccccg cttaaaaatt ggtatcagag 3180

ccatgaatag gtctatgacc aaaactcaag aggataaaac ctcaccaaaa tacgaaagag 3240

ttcttaactc taaagataaa agatctttca agatcaaaac tagttccctc acaccggtga 3300

cggggat 3307

<210> 7
<211> 4259

<212>  DNA
<213>  artificial

<220>
<223>  proVirBSV - intAtFAD2 - EcGUS - terVirCaMV


<220>
<221>  promoter
<222>  (1)..(475)
<223>  BSV promoter

<220>
<221>  Intron
<222>  (497)..(1627)
<223>  FAD 2 intron

<220>
<221>  Coding_sequence
<222>  (1650)..(3455)
<223>  GUS protein

<220>
<221>  misc_feature
<222>  (3513)..(3514)
<223>  n is a, c, g, or t

<220>
<221>  terminator
<222>  (3525)..(4259)
<223>  GUS protein

<400>  7
tctagatctg ctggatatca gtaatgatga ctgaagcgga agtggcggac ccctaccacg      60

tgttgatacc aaccggtgtg aagactgata agatgcggag tgagctggat accactcact     120

ttatgtaaag aggagacaaa gtataatgtc tctttatttt aagtttgtcg gtgtgcgttg     180

tctagtcacg cacgatgacc tttagtgact ttgcaggatt cttacgcaaa gttgttaggc     240

cagagacatg tgatgatgct tatctgcatt attggtggat gccacctaac gatgccagaa     300

agctccacaa ctctctatat aaggagcctt gtattcaggt tgcaaacacg caccacaacg     360

cgagtttact cctgatttga gaaataaaaa cttctgtgct tgaaacacac tttgtgcgag     420

ttcactttgt gcgagtagag cgcaagatcc tagttccgcg agcgtagacc cgtctgtcga     480

cggatccccg ggccaggtcc gtcgcttctc ttccatttct tctcattttc gattttgatt     540

cttatttctt tccagtagct cctgctctgt gaatttctcc gctcacgata gatctgctta     600

tactccttac attcaacctt agatctggtc tcgattctct gtttctctgt ttttttcttt     660

tggtcgagaa tctgatgttt gtttatgttc tgtcaccatt aataataatg aactctctca     720

ttcatacaat gattagtttc tctcgtctac aaaacgatat gttgcatttt cacttttctt     780

ctttttttct aagatgattt gctttgacca atttgtttag atctttattc tattttattt     840

tctggtgggt tggtggaaat tgaaaaaaaa aaaaacagca taaattgtta tttgttaatg 900

tattcatttt ttggctattt gttctgggta aaaatctgct tctactattg aatctttcct 960

ggatttttta ctcctattgg gtttttatag taaaaataca taataaaagg aaaacaaaag 1020

ttttatagat tctcttaaac cccttacgat aaaagttgga atcaaaataa ttcaggatca 1080

gatgctcttt gattgattca gatgcgatta cagttgcatg gcaaattttc tagatccgtc 1140

gtcacatttt attttctgtt taaatatcta aatctgatat atgatgtcga caaattctgg 1200

tggcttatac atcacttcaa ctgttttctt ttggctttgt ttgtcaactt ggttttcaat 1260

acgatttgtg atttcgatcg ctgaattttt aatacaagca aactgatgtt aaccacaagc 1320

aagagatgtg acctgcctta ttaacatcgt attacttact actagtcgta ttctcaacgc 1380

aatcgttttt gtatttctca cattatgccg cttctctact ctttattcct tttggtccac 1440

gcattttcta tttgtggcaa tccctttcac aacctgattt cccactttgg atcatttgtc 1500

tgaagactct cttgaatcgt taccacttgt ttcttgtgca tgctctgttt tttagaatta 1560

atgataaaac tattccatag tcttgagttt tcagcttgtt gattcttttg cttttggttt 1620

tctgcagaaa cccgggtggt cagtccctta tgttacgtcc tgtagaaacc ccaacccgtg 1680

aaatcaaaaa actcgacggc ctgtgggcat tcagtctgga tcgcgaaaac tgtggaattg 1740

atcagcgttg gtgggaaagc gcgttacaag aaagccgggc aattgctgtg ccaggcagtt 1800

ttaacgatca gttcgccgat gcagatattc gtaattatgc gggcaacgtc tggtatcagc 1860

gcgaagtctt tataccgaaa ggttgggcag gccagcgtat cgtgctgcgt ttcgatgcgg 1920

tcactcatta cggcaaagtg tgggtcaata atcaggaagt gatggagcat cagggcggct 1980

atacgccatt tgaagccgat gtcacgccgt atgttattgc cgggaaaagt gtacgtatca 2040

ccgtttgtgt gaacaacgaa ctgaactggc agactatccc gccgggaatg gtgattaccg 2100

acgaaaacgg caagaaaaag cagtcttact tccatgattt ctttaactat gccggaatcc 2160

atcgcagcgt aatgctctac accacgccga acacctgggt ggacgatatc accgtggtga 2220

cgcatgtcgc gcaagactgt aaccacgcgt ctgttgactg gcaggtggtg gccaatggtg 2280

atgtcagcgt tgaactgcgt gatgcggatc aacaggtggt tgcaactgga caaggcacta 2340

gcgggacttt gcaagtggtg aatccgcacc tctggcaacc gggtgaaggt tatctctatg 2400

aactgtgcgt cacagccaaa agccagacag agtgtgatat ctacccgctt cgcgtcggca 2460

tccggtcagt ggcagtgaag ggccaacagt tcctgattaa ccacaaaccg ttctacttta 2520

ctggctttgg tcgtcatgaa gatgcggact tacgtggcaa aggattcgat aacgtgctga 2580

tggtgcacga ccacgcatta atggactgga ttggggccaa ctcctaccgt acctcgcatt 2640

acccttacgc tgaagagatg ctcgactggg cagatgaaca tggcatcgtg gtgattgatg 2700

aaactgctgc tgtcggcttt aacctctctt taggcattgg tttcgaagcg ggcaacaagc 2760

cgaaagaact gtacagcgaa gaggcagtca acggggaaac tcagcaagcg cacttacagg 2820

cgattaaaga gctgatagcg cgtgacaaaa accacccaag cgtggtgatg tggagtattg 2880

ccaacgaacc ggatacccgt ccgcaagtgc acgggaatat ttcgccactg gcggaagcaa 2940

cgcgtaaact cgacccgacg cgtccgatca cctgcgtcaa tgtaatgttc tgcgacgctc 3000

acaccgatac catcagcgat ctctttgatg tgctgtgcct gaaccgttat tacggatggt 3060

atgtccaaag cggcgatttg gaaacggcag agaaggtact ggaaaaagaa cttctggcct 3120

ggcaggagaa actgcatcag ccgattatca tcaccgaata cggcgtggat acgttagccg 3180

ggctgcactc aatgtacacc gacatgtgga gtgaagagta tcagtgtgca tggctggata 3240

tgtatcaccg cgtctttgat cgcgtcagcg ccgtcgtcgg tgaacaggta tggaatttcg 3300

ccgattttgc gacctcgcaa ggcatattgc gcgttggcgg taacaagaaa gggatcttca 3360

ctcgcgaccg caaaccgaag tcggcggctt ttctgctgca aaaacgctgg actggcatga 3420

acttcggtga aaaaccgcag cagggaggca aacaatgaat caacaactct cctggcgcac 3480

catcgtcggc tacagcctcg ggaattgcta ccnntctaga attcggctga aatcaccagt 3540

ctctctctac aaatctatct ctctctattt tctccataaa taatgtgtga gtagtttccc 3600

gataagggaa attagggttc ttatagggtt tcgctcatgt gttgagcata taagaaaccc 3660

ttagtatgta tttgtatttg taaaatactt ctatcaataa aatttctaat tcctaaaacc 3720

aaaatccagt actaaaatcc agatctccta aagtccctat agatctttgt cgtgaatata 3780

aaccagacac gagacgacta aacctggagc ccagacgccg ttcgaagcta gaagtaccgc 3840

ttaggcagga ggccgttagg gaaaagatgc taaggcaggg ttggttacgt tgactccccc 3900

gtaggtttgg tttaaatatg atgaagtgga cggaaggaag gaggaagaca aggaaggata 3960

aggttgcagg ccctgtgcaa ggtaagaaga tggaaatttg atagaggtac gctactatac 4020

ttatactata cgctaaggga atgcttgtat ttatacccta taccccctaa taacccctta 4080

tcaatttaag aaataatccg cataagcccc cgcttaaaaa ttggtatcag agccatgaat 4140

aggtctatga ccaaaactca agaggataaa acctcaccaa aatacgaaag agttcttaac 4200

tctaaagata aaagatcttt caagatcaaa actagttccc tcacaccggt gacggggat 4259

<210> 8
<211> 4448
<212> DNA
<213> artificial

<220>
<223> proTaHMWG - intAtFAD2 - EcGUSintStLS1 - terVirCaMV

<220>
<221>  promoter
<222>  (1)..(418)
<223>  TaHMWG promoter

<220>
<221>  Intron
<222>  (467)..(1601)
<223>  FAD 2 intron

<220>
<221>  Coding_sequence
<222>  (1638)..(3632)
<223>  GUS protein

<220>
<221>  terminator
<222>  (3711)..(4448)
<223>  CAMV terminator

<400>  8
   agctttgagt ggccgtagat ttgcaaaagc aatggctaac agacacatat tctgccaaac      60
   cccaagaagg ataatcactt ttcttagata aaaaagaaca gaccaatata caaacatcca     120
   cacttctgca aacaatacat cagaactagg attacgccga ttacgtggct ttagcagact     180
   gtccaaaaat ctgttttgca aagctccaat tgctccttgc ttatccagct tcttttgtgt     240
   tggcaaactg cgcttttcca accgattttg ttcttctcgc gctttcttct taggctaaac     300
   aaacctcacc gtgcacgcag ccatggtcct gaaccttcac ctcgtcccta taaaagccta     360
   gccaaccttc acaatcttat catcacccac aacaccgagc accacaaact agagatccga     420
   attaattctc gcggccgctc tagaactagt ggatcccccg ggccaggtcc gtcgcttctc     480
   ttccatttct tctcattttc gattttgatt cttatttctt tccagtagct cctgctctgt     540
   gaatttctcc gctcacgata gatctgctta tactccttac attcaacctt agatctggtc     600
   tcgattctct gtttctctgt ttttttcttt tggtcgagaa tctgatgttt gtttatgttc     660
   tgtcaccatt aataataatg aactctctca ttcatacaat gattagtttc tctcgtctac     720
   aaaacgatat gttgcatttt cacttttctt cttttttctt aagatgattt gctttgacca     780
   atttgtttag atctttattc tattttattt tctggtgggt tggtggaaat tgaaaaaaaa     840
   aaaaacagca taaattgtta tttgttaatg tattcatttt ttggctattt gttctgggta     900
   aaaatctgct tctactattg aatctttcct ggatttttta ctcctattgg gtttttatag     960
   taaaaataca taataaaagg aaaacaaaag ttttatagat tctcttaaac cccttacgat    1020
   aaaagttgga atcaaaataa ttcaggatca gatgctcttt gattgattca gatgcgatta    1080
   cagttgcatg gcaaattttc tagatccgtc gtcacatttt attttctgtt taaatatcta    1140
   aatctgatat atgatgtcga tcgacaaatt ctggtggctt atacatcact tcaactgttt 1200
   tcttttggct ttgtttgtca acttggtttt caatacgatt tgtgatttcg atcgctgaat 1260
   tttaatacaa agcaaactga tgttaaccac aagcaagaga tgtgacctgc cttattaaca 1320
   tcgtattact tactactagt cgtattctca acgcaatcgt ttttgtattt ctcacattat 1380
   gccgcttctc tactctttat tccttttggt ccacgcattt tctatttgtg gcaatccctt 1440
   tcacaacctg atttcccact ttggatcatt tgtctgaaga ctctcttgaa tcgttaccac 1500
   ttgtttcttg tgcatgctct gttttttaga attaatgata aaactattcc atagtcttga 1560
   gttttcagct tgttgattct tttgcttttg gttttctgca gaaacccggg ctgcaggaat 1620
   tcgatatcaa gcttcccatg gtacgtcctg tagaaacccc aacccgtgaa atcaaaaaac 1680
   tcgacggcct gtgggcattc agtctggatc gcgaaaactg tggaattgat cagcgttggt 1740
   gggaaagcgc gttacaagaa agccgggcaa ttgctgtgcc aggcagtttt aacgatcagt 1800
   tcgccgatgc agatattcgt aattatgcgg gcaacgtctg gtatcagcgc gaagtcttta 1860
   taccgaaagg ttgggcaggc cagcgtatcg tgctgcgttt cgatgcggtc actcattacg 1920
   gcaaagtgtg ggtcaataat caggaagtga tggagcatca gggcggctat acgccatttg 1980
   aagccgatgt cacgccgtat gttattgccg ggaaaagtgt acgtaagttt ctgcttctac 2040
   ctttgatata tatataataa ttatcattaa ttagtagtaa tataatattt caaatatttt 2100
   tttcaaaata aaagaatgta gtatatagca attgcttttc tgtagtttat aagtgtgtat 2160
   attttaattt ataacttttc taatatatga ccaaaatttg ttgatgtgca ggtatcaccg 2220
   tttgtgtgaa caacgaactg aactggcaga ctatcccgcc gggaatggtg attaccgacg 2280
   aaaacggcaa gaaaaagcag tcttacttcc atgatttctt taactatgcc ggaatccatc 2340
   gcagcgtaat gctctacacc acgccgaaca cctgggtgga cgatatcacc gtggtgacgc 2400
   atgtcgcgca agactgtaac cacgcgtctg ttgactggca ggtggtggcc aatggtgatg 2460
   tcagcgttga actgcgtgat gcggatcaac aggtggttgc aactggacaa ggcactagcg 2520
   ggactttgca agtggtgaat ccgcacctct ggcaaccggg tgaaggttat ctctatgaac 2580
   tgtgcgtcac agccaaaagc cagacagagt gtgatatcta cccgcttcgc gtcggcatcc 2640
   ggtcagtggc agtgaagggc caacagttcc tgattaacca caaaccgttc tactttactg 2700
   gctttggtcg tcatgaagat gcggacttac gtggcaaagg attcgataac gtgctgatgg 2760
   tgcacgacca cgcattaatg gactggattg gggccaactc ctaccgtacc tcgcattacc 2820
   cttacgctga agagatgctc gactgggcag atgaacatgg catcgtggtg attgatgaaa 2880
   ctgctgctgt cggctttaac ctctctttag gcattggttt cgaagcgggc aacaagccga 2940
   aagaactgta cagcgaagag gcagtcaacg gggaaactca gcaagcgcac ttacaggcga 3000
   ttaaagagct gatagcgcgt gacaaaaacc acccaagcgt ggtgatgtgg agtattgcca 3060
   acgaaccgga tacccgtccg caagtgcacg ggaatatttc gccactggcg gaagcaacgc 3120
   gtaaactcga cccgacgcgt ccgatcacct gcgtcaatgt aatgttctgc gacgctcaca 3180
   ccgataccat cagcgatctc tttgatgtgc tgtgcctgaa ccgttattac ggatggtatg 3240
   tccaaagcgg cgatttggaa acggcagaga aggtactgga aaaagaactt ctggcctggc 3300
   aggagaaact gcatcagccg attatcatca ccgaatacgg cgtggatacg ttagccgggc 3360
   tgcactcaat gtacaccgac atgtggagtg aagagtatca gtgtgcatgg ctggatatgt 3420
   atcaccgcgt ctttgatcgc gtcagcgccg tcgtcggtga acaggtatgg aatttcgccg 3480
   attttgcgac ctcgcaaggc atattgcgcg ttggcggtaa caagaaaggg atcttcactc 3540
   gcgaccgcaa accgaagtcg gcggcttttc tgctgcaaaa acgctggact ggcatgaact 3600
   tcggtgaaaa accgcagcag ggaggcaaac aatgaatcaa caactctcct ggcgcaccat 3660
   cgtcggctac agcctcggtg acgtcggatc ccccgggctg caggaattcg gtacgctgaa 3720
   atcaccagtc tctctctaca aatctatctc tctctatttt ctccataaat aatgtgtgag 3780
   tagtttcccg ataagggaaa ttagggttct tatagggttt cgctcatgtg ttgagcatat 3840
   aagaaaccct tagtatgtat ttgtatttgt aaaatacttc tatcaataaa atttctaatt 3900
   cctaaaacca aaatccagta ctaaaatcca gatctcctaa agtccctata gatctttgtc 3960
   gtgaatataa accagacacg agacgactaa acctggagcc cagacgccgt tcgaagctag 4020
   aagtaccgct taggcaggag gccgttaggg aaaagatgct aaggcagggt tggttacgtt 4080
   gactcccccg taggtttggt ttaaatatga tgaagtggac ggaaggaagg aggaagacaa 4140
   ggaaggataa ggttgcaggc cctgtgcaag gtaagaagat ggaaatttga tagaggtacg 4200
   ctactatact tatactatac gctaagggaa tgcttgtatt tataccctat accccctaat 4260
   aaccccttat caatttaaga aataatccgc ataagccccc gcttaaaaat tggtatcaga 4320
   gccatgaata ggtctatgac caaaactcaa gaggataaaa cctcaccaaa atacgaaaga 4380
   gttcttaact ctaaagataa aagatctttc aagatcaaaa ctagttccct cacaccggtg 4440
   acggggat 4448

<210> 9
<211> 25
<212> DNA
<213> Artificial

<220>
<223> forward primer

<400> 9

gggccaggtc cgtcgcttct cttcc                                              25

<210>  10
<211>  24
<212>  DNA
<213>  artificial

<220>
<223>  reverse primer

<400>  10
gggtttctgc agaaaaccaa aagc                                               24

<210>  11
<211>  19
<212>  DNA
<213>  artificial

<220>
<223>  forward primer

<400>  11
gatgctctag atctgctgg                                                     19

<210>  12
<211>  29
<212>  DNA
<213>  artificial

<220>
<223>  reverse primer

<400>  12
ggccatggct tgctctgata ccagacggg                                         29

## Description

### BACKGROUND OF THE INVENTION

Efficient transformation systems are available in plants, in particular in monocot species especially those of economic importance such as maize, wheat, barley and rice. It is now possible to contemplate the introduction of a range of traits by such technique. The most widely used techniques are the delivery of naked DNA by micro projectile bombardment or the use of Agrobacterium as a vector. In both cases the gene of interest and associated sequences are introduced into a suitable target plant tissue and become stably integrated into the plant genome. The target tissue has the potential to regenerate into a whole, fully-fertile plant.

In order for the introduced gene to be expressed, it must be driven by a promoter. Promoters can be of different types; constitutive or tissue and temporally specific. Widely use promoters include those derived from virus and bacteria; including Cauliflower Mosaic Virus 35S and octopine and nopaline synthase and RuBISCo, actin and ubiquitin from plant species.

The most widely used promoters are constitutive because they are generally used to control the selectable marker gene and can also be used to drive the gene of interest.

Although suitable promoters for monocot species are known, insufficient numbers are available for use in biotechnology applications. Transformation may require that one promoter drives the selectable marker and a separate promoter drives the gene of interest.

It is indeed undesirable to use the same promoter for both genes for several reasons. Initially the cloning and construct preparation becomes problematical. Sequence duplications can lead to gene deletions and other corruptions which would result in elimination of gene expression. Sequence duplication subsequently *in planta* can result in gene silencing. In a longer term consideration of breeding programmes, either gene stacking by retransformation or crossing of existing transgenic lines, the lack of choice of promoters will become increasingly limiting.

Strong promoters are preferred as the efficiency of production of useful transgenic plants is increased, in two ways. Firstly more plants are initially selected in tissue culture due to sufficient expression of the selectable marker to survive the selection conditions and secondly more plants thus selected will have a good phenotype resulting from the expression of the gene or genes of interest. In order to increase the number of suitable promoters it was discovered that the strength of a promoter can be increased by use of an intron.

Introns interrupt the coding regions of Eukaryotic genes and are removed post-transcription by the process of splicing. The expression of genes in animal cells has been known to be intron dependant since the late 1970s but the first demonstration of the effect of the inclusion of an intron in a plant species was not until 1987 (Callis et al , Genes and Development 1: 1183-1200).

The most widely used introns are the first introns of rice Actin and maize Ubiquitin, used in combination with the natural promoters. These promoters were the first strong promoters identified for monocot species. Rice actin was initially identified as a suitable candidate gene for provision of a strong constitutive promoter because actin is a fundamental and essential component of the eukaryotic cell and cytoskeleton. A rice actin gene was identified that encoded a transcript that was abundant in all rice tissues at all developmental stages (McElroy et al 1990.) Further characterisation revealed that the presence of the first intron was essential for the efficient function of the promoter *per se*. Similarly the maize ubiquitin promoter was identified as having a high level constitutive activity dependant on the presence of its own first intron (Morris et al 1993).

The enhancing effect of the rice actin first intron and the maize ubiquitin first intron can also be combined with heterologous promoter sequences which alone have minimal activity in monocot species. (Vain et al, 1996))

A limited number of plant introns have been identified that can be combined with suboptimal promoters to create novel promoters sufficiently strong to use for biotechnological applications. Early comparisons of a range of introns revealed that not all have a significantly enhancing activity in combination with a suboptimal promoter. (Vain et al, 1996)

Nevertheless, intron duplication is undesirable for same reasons that promoter duplication should be avoided.

There is therefore a continuing need to identify new suitable introns to enhance expression in plants, and especially in monocot transformation systems. These introns need to be able to increase the level of expression when combined with promoters.

At the present time there are very few such introns identified and this ultimately limits the number of genetic transformations that can be carried out in a given monocot species.

The invention now provides a new intron that is usable for increasing expression of an heterologous gene in a plant cell, and in particular a monocot plant cell.

### SUMMARY OF THE INVENTION

The inventors have indeed surprisingly observed that presence of the first intron of the FAD2 gene, which was believed to have no effect on expression, can effectively enhance the efficiency of a promoter.

The FAD2 gene is the gene coding for the microsomal omega-6 fatty acid desaturase (FAD2/delta-12 desaturase), and its sequence is well known by the person skilled in the art, with the availability of the sequence of the chromosome 3 *Arabidopsis thaliana* genome.

Other FAD2 genes have been identified (for example *Brassica oleracea*, represented by AF181726 in GenBank, soybean with GenBank protein accession number P48628, *Brassica napus*, GenBank protein accession number P48627...). The identification of the first introns of FAD2 genes of other species is within the skill of the person in the art, by the differences between the cDNA and genomic sequences.

### FIGURES

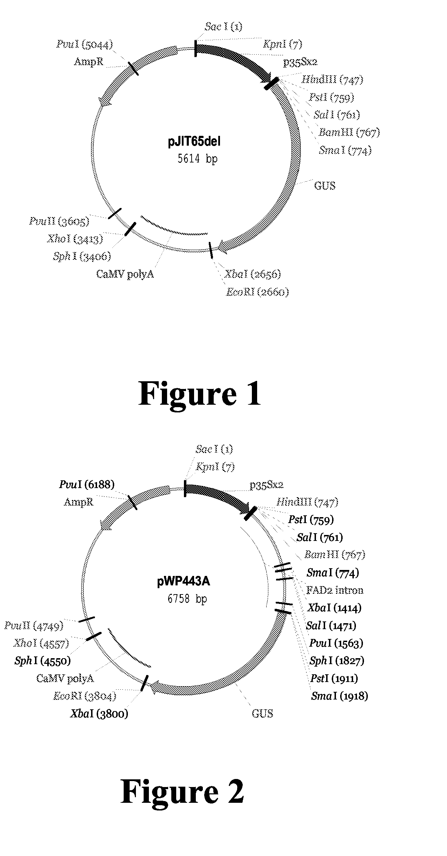

**Figure 1:** Schematic representation of pJIT65del
**Figure 2:** Schematic representation of pWP443A

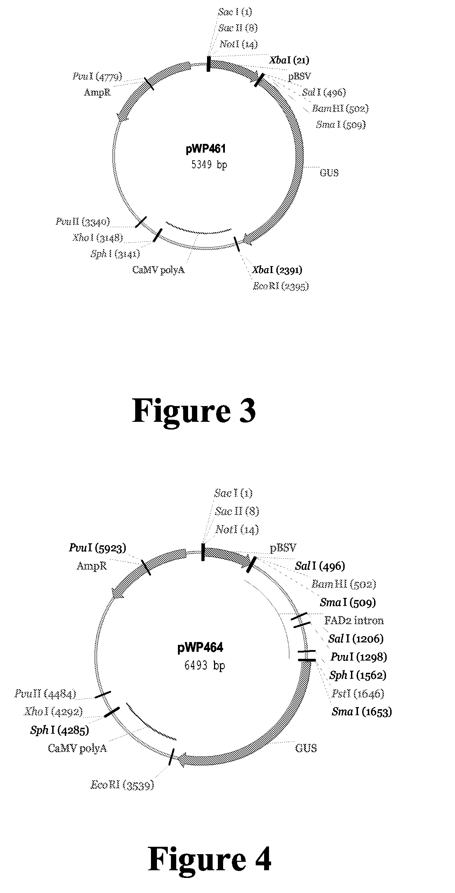

**Figure 3 :** Schematic representation of pWP461
**Figure 4:** Schematic representation of pWP464

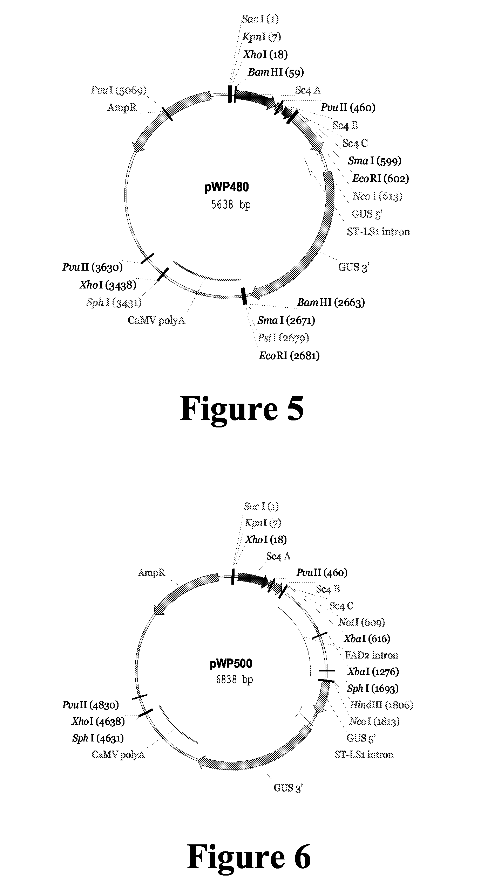

**Figure 5:** Schematic representation of pWP480
**Figure 6 :** Schematic representation of pWP500

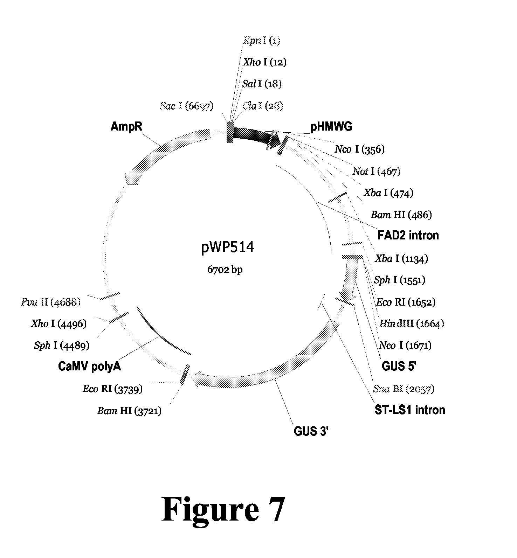

**Figure 7 :** Schematic representation of pWP514
**Figure 8 :** Transient expression comparison in wheat. Embryos bombarded with 3 promoters with and without FAD2 intron.
**Figure 9 :** Transient expression comparison in barley a) embryos bombarded with pWP480 b) embryos bombarded with pWP500

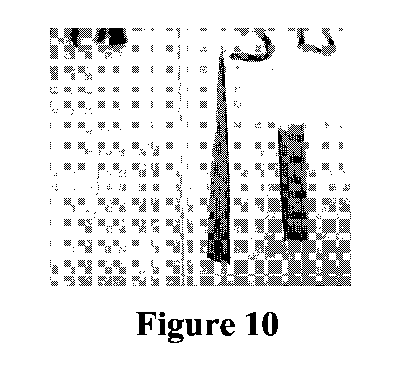

**Figure 10:** Leaves from wheat stably transformed wheat with a) pBSV without FAD2 intron, or b) pBSV with FAD2 intron.

### DETAILED DESCRIPTION OF THE INVENTION

The invention thus relates to a genetic construct for the expression of a polypeptide in a plant cell, comprising:
(a) a promoter operative within said plant cell,
(b) all or part of the first intron of a FAD2 gene,
(c) a nucleotide sequence operably linked to said promoter, wherein said nucleotide sequence encodes said polypeptide,
(d) a polyadenylation site (PAA) operably linked to said nucleotide sequence.

A "promoter operative in a plant cell" is intended to mean a sequence that direct the initiation of transcription in a plant cell. It can derive from one or multiple sources, be natural or synthetic.

In the preferred embodiment, the genetic construct of the invention is such that said FAD2 gene is from *Arabidopsis thaliana* (GenBank AJ271841), wherein said first intron is represented by SEQ ID N° 1, or as indicated in SEQ ID N° 3, 5, 7 and 8. it is also envisaged that said FAD 2 intron has 80, 90 or 95 % identity with SEQ ID N° 1, over at least 500 or 1000 nucleotides.

Nevertheless, other FAD2 first introns from other species may also be used, such as from maize, wheat, barley, rape, canola, sunflower, potato...

In the context of the present invention, "all or part of the first intron of a FAD2 gene" is to be considered as the full sequence of the first intron of a FAD 2 gene, or a fragment comprising at least 200, 500 or 1000 nucleotides of said sequence.

The promoter could be any promoter, as described above. In a preferred embodiment, said promoter is a tissue-specific promoter. In another embodiment, said promoter is a constitutive promoter.

Promoter sequences of genes which are expressed naturally in plants can be of plant, bacterial or viral origin. Suitable constitutive promoters include but are not restricted to octopine synthase, nopaline synthase, mannopine synthase derived from the T-DNA of *Agrobacterium tumefaciens*; CaMV35S and CaMV19S from Cauliflower Mosaic Virus; rice actin, maize ubiquitin and histone promoters from plant species.

Tissue specific promoters include but are not restricted to rolC from *Agrobacterium rhizogenes;* RTBV from Rice Tungro Bacilliform Virus; LMW and HMW glutenin from wheat; alcohol dehydrogenase, waxy, zein from maize and, AoPR1 from Asparagus.

All these promoters (and others) are well described in the art.

Whilst the most widely used promoters are constitutive or tissue specific it is also possible to contemplate the use of inducible promoters such as PinII or AoPRT-L (WO 99/66057); more specifically light inducible including RUBISCO small subunit and chlorophyll a/b binding protein from a range of species and synthetic promoters eg EMU (US19900525866).

The genetic construct according to the invention is introduced within the plant cell on a vector. In an embodiment, said vector is maintained as an episomal vector within the plant cell. In the preferred embodiment, said vector is capable of integration within the genome of said plant cell. Integration of a genetic construct within a plant cell is performed using methods known by those skilled in the art, for example transformation by Agrobacterium, or gun bombardment.

Agrobacterium tumefaciens has been widely and efficiently used to transform numerous species dicots and including monocots such as maize, rice, barley and wheat (WO 00/63398). Alternative gene transfer and transformation methods include protoplast transformation through calcium, polyethylene glycol or electroporation mediated uptake of naked DNA. Additional methods include introduction of DNA into intact cells or regenerable tissues by microinjection, silicon carbide fibres or, most widely, microprojectile bombardment. All these methods are now well known in the art.

The invention also relates to a method for enhancing the expression of a polypeptide in a plant cell, comprising the step of introducing the genetic construct of the invention within said plant cell, for example using the methods as mentioned above. Expression is enhanced as compared to expression obtained when the FAD2 intron is not present within the construct.

Expression relates to transcription and translation of a gene so that a protein is made.

In another aspect, the invention relates to a method for increasing the expression rate of a gene in a plant cell, said method comprising the steps of:
(a) inserting the first intron of the FAD 2 gene between said promoter and the translation start of said gene, thereby forming an intron-modified gene; and
(b) introducing said intron-modified gene into a plant cell such that said gene is expressed under the control of said intron in said plant cell, whereby the expression rate of said gene is increased as compared to an unmodified gene.

The invention also relates to a method as mentioned above wherein said intron has a nucleotide sequence as set forth in SEQ ID N° 1 or functionally equivalent duplications or modifications in length or minor sequence variations that do not significantly affect function, which is expression enhancing. It is well within the skills of a person skilled in the art to modify the FAD 2 intron sequence and test new constructs for their ability to improve expression of a gene, various protocols in the art, and in particular the ones described in the examples.

In another aspect, the invention relates to the use of all or part of the first intron of the FAD2 gene for enhancing expression of a polypeptide in a plant cell.

In another aspect, the FAD2 first intron may be used to coNvert a tissue specific promoter into a constitutive promoter. Fore example, the strong seed specific HMW glutenin promoter is converted into a strong/ useful promoter active in many tissue types, when used with the FAD 2 first intron.

### EXAMPLES

All DNA modifications and digestions were performed using enzymes according to the manufacturers' instructions and following protocols described in Sambrook and Russell, 2001; Molecular Cloning, A Laboratory Manual.

### Example 1. Cloning of FAD 2 intron

The first intron of the FAD 2 gene was obtained by the polymerase chain reaction (PCR) from *Arabidopsis thaliana* genomic DNA. The following pair of primers were used:

GGGCCAGGTCCGTCGCTTCTCTTCC FADfor (SEQ ID N° 9)

GGGTTTCTGCAGAAAACCAAAAGC FADrev (SEQ ID N° 10)

A standard PCR reaction was carried out, with amplification under the following conditions: 30 cycles of 97°C for 30 seconds, 57°C for 30 seconds and 74°C for 1 minute 40 seconds. The reaction products were separated by agarose gel electrophoresis.

The desired 1146 bp product was excised from the gel and ligated into the *Sma*I restriction site of pTZ18 (standard cloning vector; Pharmacia) to create pWP430. The fidelity of the clone was confirmed by sequencing.

### Example 2. Preparation of GUS constructs

Several promoters, which are known in the field of plant biotechnology were identified, to be tested with and without the FAD2 intron. These included but were not limited to Cauliflower Mosaic Virus 35S (Odell et al,Nature. 1985 Feb 28-Mar 6;313(6005):810-2), Banana Streak Virus promoter of ORF1 (WO 99/43836), a member of the Plant Expression (PLEX) promoter family (EP 785999; US 6211431) and HMW glutenin promoter. Constructs were prepared from well-characterised genetic elements and cloned into widely used plasmid vectors.

### 35S: pJIT65del

The 35S promoter was cloned in a pUC vector, driving the GUS gene (Jefferson et al, PNAS 83: 8447-8451, 1986), to obtain plasmid pJIT65. This plasmid was modified by digestion with *Xba*I and *EcoR*I to removing intervening *BamH*I and *Sma*I sites. This modified version was named as pJIT65del.

### 35S + FAD2 intron:pWP443A

The FAD2 intron was inserted between the 35S promoter and the GUS gene, specifically into the 5'UTR, by digesting pWP430 with *Sma*I and ligating the intron into *Sma*I digested pJIT65del, to create pWP443A.

### BSV: pWP461

The BSV promoter was cloned into the *EcoR*V site of Bluescript KS to create pWP453B. The following primers were used:

GATGCTCTAGATCTGCTGG BSVFor (SEQ ID N° 11)

GGCCATGGCTTGCTCTGATACCAGACGGG     BSVRev (SEQ ID N° 12)

pWP453B was digested with *Sst*I and *Sal*I to isolate the BSV promoter fragment which was then ligated into *Sst*I and *Sal*I digested JIT65del, thus replacing the 35S promoter to create pWP461.

### BSV + FAD2 intron:pWP464

To create the combination of the BSV promoter with the FAD2 intron the 35S promoter of pWP443A was replaced by the BSV promoter from pWP461. Both plasmids were digested with SstI and BamHI and fragments ligated to produce pWP464.

### Sc4:pWP480

A previously prepared construct, pKH2, having the Sc4 promoter driving the GUS gene, with the inclusion of the rice actin first intron in the 5'UTR, was digested with SstII and NcoI to remove the actin intron and create pWP480.

### Sc4 +FAD2 intron:pWP 500

Similarly to the creation of pWP464, to combine the Sc4 promoter with the FAD2 intron the 35S promoter of pWP443A was replaced by the Sc4 promoter from pWP480 using suitable digests.

### HMWG + intron: pWP514

A pUC clone of the HMWG promoter driving the GUS gene (Glu-1Dx5-GUS2) was obtained as described in Halford et al 1989 (Plant Science 62, 207-16). The FAD2 intron was inserted between the HMWG promoter and the GUS gene, specifically into the 5'UTR, to create pWP514.

### Example 3 Transient expression in wheat

### a) Initial assessment of functioning of constructs

A range of plant tissues and calli were bombarded with particles coated with one of the four promoter constructs including the FAD2 intron, according to methods known in the art. The tissues were immersed in a solution of the histochemical substrate. X-glucuronide, and a subjective assessment was made of the activity of the construct. In each case a significant number of blue spots were observed on each of the tissues tested.

| | TRANSIENT EXPRESSION |
|---|---|
| pWP443A | Strong expression on embryo, leaf and callus |
| pWP500 | Strong expression on embryo, leaf and callus |
| pWP464 | Strong expression on embryo, leaf and callus |
| pWP514 | Good expression on embryo, leaf and endosperms |

It is especially worthy of note that there was significant expression in leaf and embryo tissues following bombardment with the HMWG construct (pWP514). The HMWG promoter is naturally strong endosperm-specific promoter. The inclusion of the FAD2 intron has converted it into a strong constitutive promoter.

### b) Comparison of constitutive promoters with and without introns

Having established that the promoters modified by the inclusion of the FAD2 intron were functioning a quantitative comparison was made between the promoters with and without the intron.

Isolated embryos were arranged in 4x4 arrays. Thirty-two embryos were bombarded with each construct.

Co-bombardment with a second construct containing the Green Fluorescent Protein (GFP) under control of the rice actin promoter, was performed. Expression of GFP can be non-destructively tested and hence the relative efficiency of each individual bombardment can be normalized to allow comparison between individual bombardments.

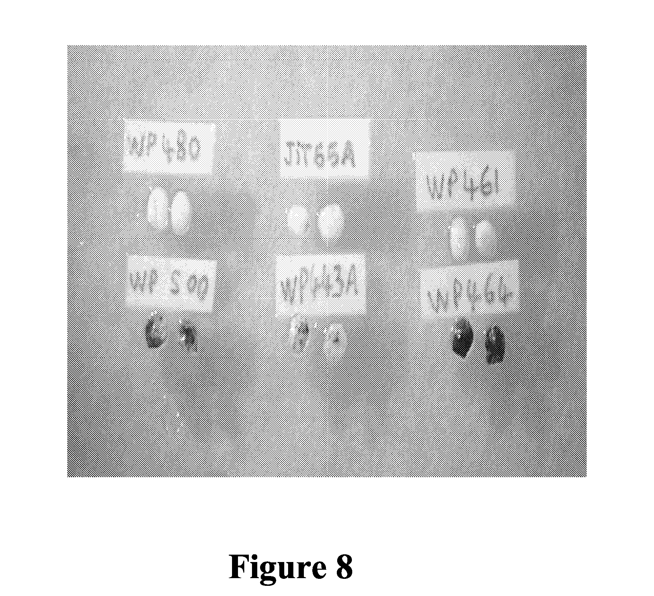

The level of expression for each construct was again visually assessed by histochemical assay for the GUS gene (figure 8) which enabled both a quantitative (counting the number of blue foci by 4x4 array), and a qualitative assessment (consideration of the size of blue foci).

The increase in expression due to inclusion of the FAD2 intron was 5.6 fold for the Sc4 promoter, 8 fold for the double 35S promoter and most remarkably 161 fold for the BSV promoter. Thus the expression of the weakest promoter (BSV) is increased to a level comparable with the maize ubiquitin promoter (pAAA) which is widely used because it is known to be very strong.

| | pSc4 | | | | pBSV | | | |
|---|---|---|---|---|---|---|---|---|
| | pWP480 | | pWP500 | | pWP461 | | pWP464 | |
| GFP | 1648 | 698 | 1501 | 1945 | 989 | 1015 | 1504 | 1384 |
| GUS | 610 | 142 | 2340 | 3101 | 36 | 14 | 5067 | 4287 |
| Note on GUS spots | Very small spots, difficult to see with the naked eye | | Big spots, well seen but no spreading nor covering | | Very small spots (two big spots on two different embryos) | | Very big, 'spreading' spots | |
| Ratio GUS/GFP | 0.37 | 0.20 | 1.55 | 1.59 | 0.03 | 0.01 | 3.36 | 3.09 |
| Average | 0.28 | | 1.57 | | 0.02 | | 3.22 | |
| Ratio with/without | 5.6 | | | | 161 | | | |

| | p35S | | | | Positive Control | |
|---|---|---|---|---|---|---|
| | pJIT65del | | pWP443A | | pAAA | |
| GFP | 470 | 922 | 711 | 687 | 1502 | 1390 |
| GUS | 93 | 296 | 1539 | 1279 | 7200 | 7200 |
| Note on GUS spots | Very small dots, sometimes not seen with the naked eye, few big ones | | Plenty of rather big spots | | Very big spots, 'spreading' all over the embryo difficult to score | |
| Ratio GUS/GFP | 0.19 | 0.32 | 2.16 | 1.86 | 4.79 | 5.17 |
| Average | 0.25 | | 2.01 | | 4.98 | |
| Ratio with/without | 8 | | | | / | |

A negative control with only gold particles yielded no spots.

### Example 3: Transient expression in barley

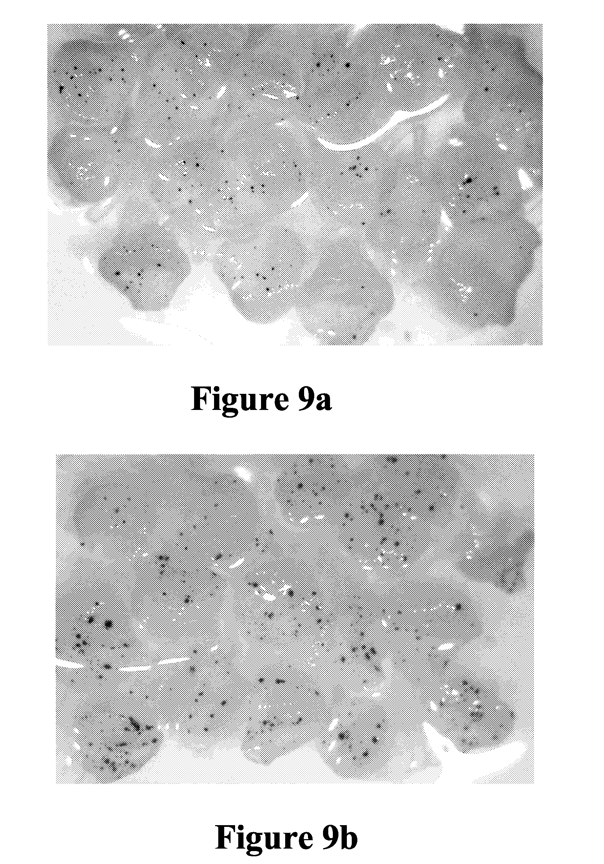

Other bombardment experiments were performed on barley embryos. The results also demonstrated that the presence of the FAD2 intron increases expression of the GUS protein in this species (figure 9). 2 plates of 16 embryos were bombarded with either pWP480 or pWP500 plasmid. The number of blue foci was scored per embryo and averaged.

Expression of co-bombarded GFP was again used to assess the relative efficiency of each individual bombardment and used to normalize and allow comparison between individual bombardments.

| | pWP480 | pWP500 |
|---|---|---|
| GFP | 63.9 | 60.7 |
| GUS | 27.00 | 92.2 |
| RatioGUS/GFP | 0.42 | 1.52 |
| Ratio +/- intron | 3.61 | |

### Example 4: Stable expression in wheat

Wheat embryos were stably transformed by bombardment (WO 98/49316).

Transgenic plants were regenerated and grown, and expression of the GUS protein was studied by X-Gluc staining for glucuronidase activity. Strong constitutive expression could be observed in a range of tissues including leaf, root, male and female floral parts and seed.

## Claims

1. A genetic construct for the expression of a polypeptide in a plant cell, comprising:
(a) a promoter operative within said plant cell,
(b) the first intron of the FAD2 gene,
(c) a nucleotide sequence operably linked to said promoter, wherein said nucleotide sequence encodes said polypeptide,
(d) a polyadenylation site (PAA) operably linked to said nucleotide sequence.

2. The genetic construct of claim 1, wherein said FAD2 gene is from Arabidopsis thaliana (GenBank AJ271841), wherein said first intron is represented by SEQ ID N° 1.

3. The genetic construct of any of claims 1 to 2, wherein said promoter is a tissue-specific promoter.

4. The genetic construct of any of claims 1 to 2, wherein said promoter is a constitutive promoter.

5. The genetic construct of any of claims 1 to 4, capable of integration within the genome of said plant cell.

6. A method for enhancing the expression of a polypeptide in a plant cell, comprising the step of introducing the genetic construct of any of claims 1 to 5 within said plant cell.

7. A method for increasing, in a plant cell, the expression rate of a gene under the control of a promoter, said method comprising the steps of:
(a) inserting the first intron of the FAD 2 gene between said promoter and the translation start of said gene, thereby forming an intron-modified gene; and
(b) introducing said intron-modified gene into a plant cell such that said gene is expressed under the control of said intron in said plant cell, whereby the expression rate of said gene is increased as compared to an unmodified gene.

8. The method of claim 6 wherein said intron has a nucleotide sequence as set forth in SEQ ID N° 1 or functionally equivalent duplications or modifications in length or minor sequence variations that do not significantly effect function.

9. The use of the first intron of the FAD2 gene for enhancing expression of a polypeptide in a plant cell.
